# EUROPEAN PATENT SPECIFICATION

(11) **EP 2 719 316 B1**
(45) Date of publication and mention of the grant of the patent: **02.08.2017**
(21) Application number: 12797664.5
(22) Date of filing: 05.06.2012
(51) Int. Cl.: A61B 1/00, A61B 5/00, A61B 5/01, A61B 17/34

(54) **INSERTION INLET FITTING**
ANSCHLUSSSTÜCK FÜR EINSATZ
ADAPTATEUR D'ENTRÉE D'INTRODUCTION

(30) Priority: 07.06.2011 JP 2011127646
(43) Date of publication of application: 16.04.2014
(73) Proprietor: Olympus Corporation, Tokyo 192-8507 (JP)
(72) Inventor: ITO, Takeshi, Hachioji-shi Tokyo 192-8512 (JP)
(74) Representative: Winter, Brandl, Fürniss, Hübner, Röss, Kaiser, Polte - Partnerschaft mbB
(86) International application number: PCT/JP2012/064455
(87) International publication number: WO 2012/169491

(56) References cited:
- WO-A1-2011/016428
- WO-A1-2011/016428
- JP-A- 5 337 127
- JP-A- 2002 028 125
- JP-A- 2009 240 405
- JP-A- 2010 022 762
- JP-U- 56 113 514
- JP-U- 56 113 514

## Description

### BACKGROUND OF THE INVENTION

### 1. Field of the Invention

The present invention relates to an insertion opening attachment that is used in combination with each of various types of endoscopes.

### 2. Description of the Related Art

In general, there is known an insertion opening attachment such as a mouthpiece that guides an inserting operation of an insertion unit of an endoscope at the time of inserting a distal end of the insertion unit of the endoscope into a human body which is an observation target. For example, JP 2002-28125 A suggests a technology that combines an insertion opening attachment with an optical sensor formed of a light emitting element and a light receiving element and detects whether a distal end of an insertion unit of an endoscope is present in an observation target after having passed through the insertion opening attachment.

A through hole configured to allow an insertion unit of an endoscope to be inserted therein is formed in a mouthpiece. An optical sensor formed of a light emitting element and a light receiving element is incorporated in an inner peripheral surface of this through hole. A reflecting portion is provided on an outer peripheral surface of the insertion unit of the endoscope. Further, in a state that a distal end of the insertion unit of the endoscope is inserted in the through hole of the mouthpiece, light emitted from the light emitting element is reflected by the reflecting portion of the insertion unit of the endoscope, and the reflected light is detected by the light receiving element. On the other hand, in a state that the insertion unit of the endoscope is not inserted in the mouthpiece, a light volume detected by the light receiving element is smaller than that when the insertion unit is inserted.

As a result, the light receiving and emitting elements of the insertion opening attachment are used for detecting whether the insertion unit of the endoscope is present inside or outside, and it is possible to execute light source control considering safety relative to a living body, e.g., emitting a laser beam only when the insertion unit of the endoscope is present in the living body.

In JP 2002-28125 A, a state is detectable, whether the insertion unit of the endoscope has passed through the mouthpiece. However, a state that the insertion unit of the endoscope is inserted in a body is not discriminated from a state that this unit is arranged outside the body, and hence the following inconvenience occurs in case of performing an inserting operation of the endoscope.

As an example of a technique at the time of inserting an endoscope, there is known a technique by which an insertion unit of the endoscope is inserted in a mouthpiece in advance, then a distal end of the insertion unit of the endoscope is inserted into a body, and thereafter the mouthpiece is attached to an endoscope insertion opening of a living body. When this technique is applied to the technology disclosed in JP 2002-28125 A, the insertion unit of the endoscope has passed through the mouthpiece even though the insertion unit of the endoscope is actually present outside the body at the time of attachment of the mouthpiece, the insertion unit of the endoscope may be possibly erroneously determined to be present in the body. Therefore, an insertion opening attachment that enables assuredly detecting the inside or the outside of a body without erroneous detection is needed.

From JP 56 113514 U a mouthpiece to be used in endoscopic examinations is known. This mouthpiece has a pressure-sensitive characteristic at its outer periphery informing an operator about a proper fit of the mouthpiece between a patient's teeth by issuing a sound signal.

US 2011/0230712 A1 discloses an anus mount tool comprising a infrared sensor measuring biological information of the patient and an encoder detecting the movement of an endoscope inserted through the anus mount tool.

### BRIEF SUMMARY OF THE INVENTION

According to an embodiment of the present invention, there is provided an insertion opening attachment that is used in combination with an endoscopic apparatus configured to observe an object inner surface of a subject and attached to an insertion opening which is an opening portion of the object before an insertion unit of the endoscopic apparatus is inserted. The insertion opening attachment has a cylindrical attachment main body that is fixed to a peripheral edge region of the opening portion of the insertion opening while being inserted in the insertion opening of the object, and the attachment main body comprises an attachment state detection unit configured to detect whether the attachment main body is attached to the insertion opening. The attachment main body furthermore comprises an insertion state detection unit configured to detect whether the insertion unit of the endoscope apparatus is inserted in the insertion opening.

### BRIEF DESCRIPTION OF THE SEVERAL VIEWS OF THE DRAWINGS

FIG. 1 is a longitudinal cross-sectional view showing an outline configuration in a state that an insertion opening attachment of an endoscope according to a first embodiment is attached to an insertion opening of a human body;
FIG. 2 is a perspective view showing an attachment main body of the insertion opening attachment of the endoscope according to the first embodiment;
FIG. 3 is a longitudinal cross-sectional view showing an attachment main body of the insertion opening attachment of the endoscope according to the first embodiment;
FIG. 4 is a schematic block diagram of an entire system of the endoscope according to the first embodiment;
FIG. 5 is a block diagram showing a connection state of a detector of the insertion opening attachment of the endoscope according to the first embodiment;
FIG. 6 is a longitudinal cross-sectional view showing a modification of the attachment main body of the insertion opening attachment of the endoscope according to the first embodiment;
FIG. 7 is a perspective view showing an outline configuration of an attachment main body of an insertion opening attachment of an endoscope according to a second embodiment;
FIG. 8 is a perspective view showing a modification of the attachment main body of the insertion opening attachment of the endoscope according to the second embodiment;
FIG. 9 is a longitudinal cross-sectional view showing an attachment main body of an insertion opening attachment of an endoscope according to a third embodiment;
FIG. 10 is a perspective view showing an attachment main body of an insertion opening attachment of an endoscope according to a fourth embodiment;
FIG. 11 is a longitudinal cross-sectional view showing an attachment main body of an insertion opening attachment of an endoscope according to a fifth embodiment; and
FIG. 12 is a perspective view showing the attachment main body of the insertion opening attachment of the endoscope according to the fifth embodiment.

### DETAILED DESCRIPTION OF THE PREFERRED EMBODIMENT

Embodiments according to the present invention will now be described hereinafter with reference to the drawings.

### [First Embodiment]

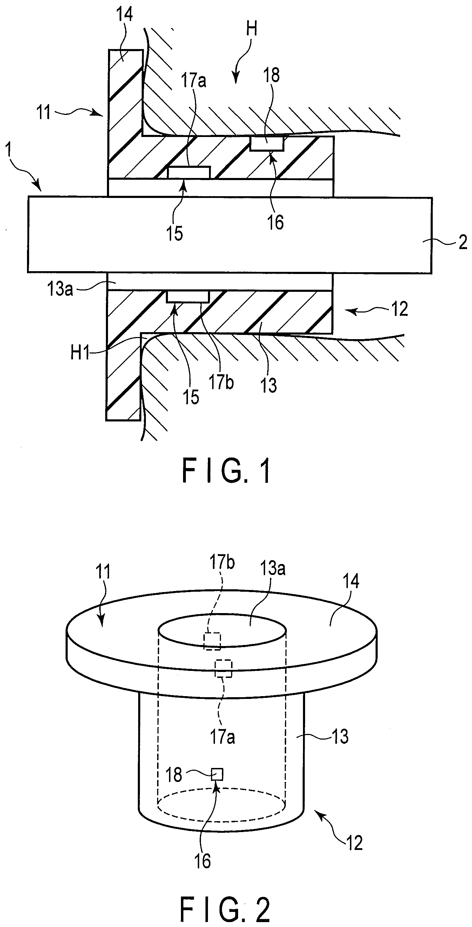

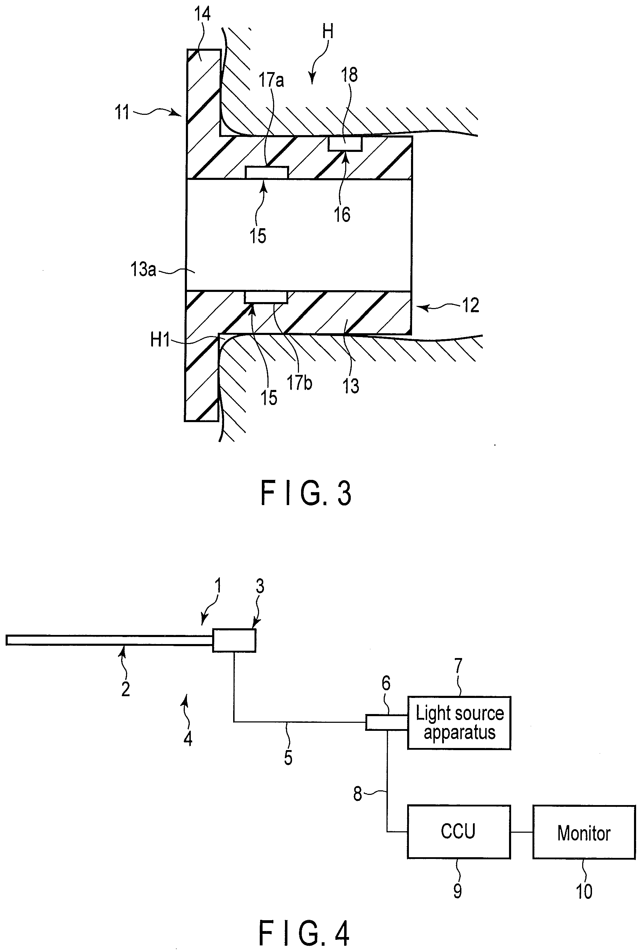

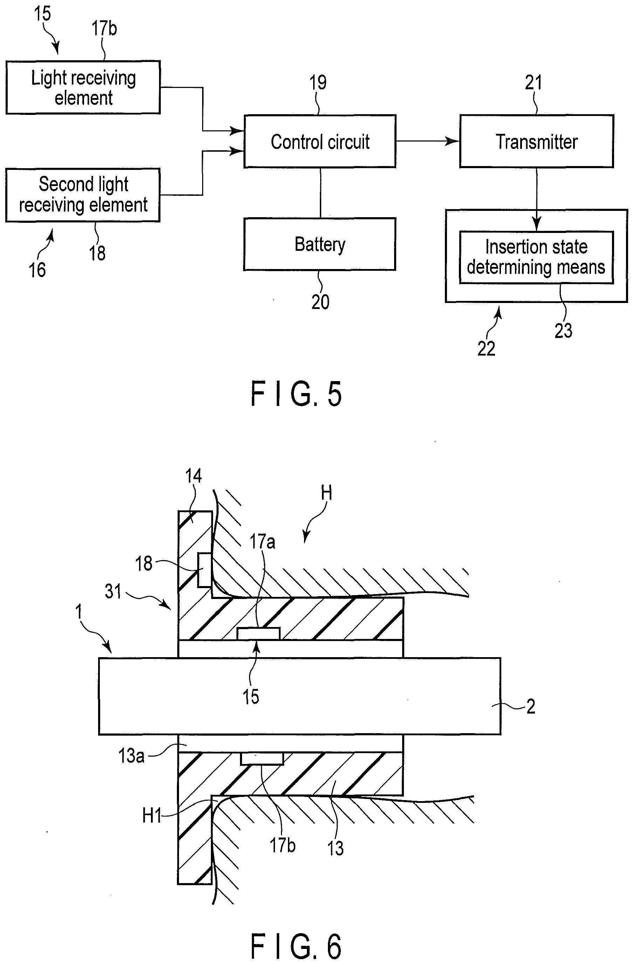

FIG. 1 and FIG. 3 show schematic cross-sectional configurations of an insertion opening attachment of an endoscope according to a first embodiment of the present invention. Similarly, FIG. 2 show an appearance configuration of the insertion opening attachment of the endoscope. FIG. 4 show a schematic block diagram of an entire system of the endoscope as a medical instrument configured to give a treatment to or observe a patient. FIG. 5 show block configurations of the insertion opening attachment of the endoscope.

The endoscope 1 has a scope unit 4 comprising an elongated insertion unit 2 that is inserted into a body cavity of a living body and an operation unit 3 that is coupled with a proximal end portion of this insertion unit 2 and operates the endoscope 1. Further, one end of a universal cord 5 is connected to the operation unit 3. The other end of this universal cord 5 is connected to a light source apparatus 7 through a connector unit 6.

Furthermore, the connector unit 6 is connected to a camera control unit (CCU) 9 through an electric cable

### 8. A monitor 10 is connected to this CCU 9.

Moreover, in the endoscope 1, illumination light supplied from the light source apparatus 7 exits from a non-illustrated illumination window provided at a distal end portion of the insertion unit 2 and illuminates the inside of a body cavity. Additionally, an observation image that is allowed to enter from a non-illustrated observation window likewise provided at the distal end portion is converted into an electrical signal by an imaging element such as CCD and supplied to the CCU 9, and an image processed by the CCU 9 is displayed in the monitor 10.

Further, in the upper digestive organ endoscope 1, for example, a human mouth H1 is determined as an insertion opening (an opening portion of an object), and the insertion unit 2 is inserted into a body. At this time, there is known a technology by which a cylindrical insertion opening attachment 11 such as a mouthpiece that guides an inserting operation of the endoscope insertion unit 2 is attached to the human mouth H1 which is the insertion opening as shown in FIG. 1 and an inserting operation of the endoscope insertion unit 2 is performed.

It is to be noted that, in this embodiment, the insertion opening attachment 11 for the upper digestive organ endoscope configured to observe a digestive organ in a human body will be taken as an example and explained. However, the insertion opening attachment 11 used in combination with the endoscope apparatus is not restricted to observation of an inner surface of an object of a subject. For example, it is possible to use not only various endoscopes used for human bodies such as a lower digestive endoscope or a laparoscope but also endoscopes for animals or industrial endoscopes for the insertion opening attachment that guides an inserting operation of each of various endoscopes.

FIGS. 1 and 2 show the insertion opening attachment 11 according to this embodiment. FIG. 1 shows a state that the insertion opening attachment 11 is disposed to the human mouth H1, and FIG. 2 is a perspective view showing appearance of the insertion opening attachment 11. This insertion opening attachment 11 has a flanged cylindrical attachment main body 12 that is fixed to a peripheral edge region of an opening portion of the human mouth H1 while being inserted in the human mouth H1.

The attachment main body 12 is constituted of a cylindrical wall portion 13 that is inserted into the human mouth H1 which is an opening of a living body H and a flange-shaped stopper portion 14 that functions as a stopper that prevents the insertion opening attachment 11 from falling into the living body.

In this embodiment, the cylindrical wall portion 13 is a cylindrical wall portion into which the insertion unit 2 can be inserted. In the following description and drawings, the cylindrical wall portion 13 is represented as a cylinder because it is simple. Further, the stopper portion 14 is formed of a discoid extended member that is provided at one end of the cylindrical wall portion 13 and extended toward the outer side in a direction crossing an inserting direction of the insertion unit 2.

The insertion opening attachment 11 is made of a material having properties that the attachment main body 12 blocks at least part of outside light. More preferably, the attachment main body 12 is made of a material having properties that block almost all of the outside light.

The insertion opening attachment 11 has an insertion state detection unit 15 that detects whether the endoscope 1 is inserted in the attachment main body 12 and an attachment state detection unit 16 having a detection unit that detects whether the insertion opening attachment 11 is attached to the human mouth H1. The insertion state detection unit 15 in this embodiment is constituted of a pair of light receiving/emitting elements arranged on an inner peripheral surface of a cylindrical inner portion 13a of the cylindrical wall portion 13 of the insertion opening attachment 11.

The pair of light receiving/emitting elements have a light emitting element 17a and a light receiving element 17b that detects light emitted from this light emitting element 17a. The light emitting element 17a and the light receiving element 17b are arranged on opposed wall surfaces (positions that are 180° apart from each other in the circumferential direction) of an inner peripheral surface of the cylindrical inner portion 13a of the cylindrical wall portion 13. In the insertion state detection unit 15, a light volume received by the light receiving element 17b varies depending on presence/absence of insertion of the endoscope insertion unit 2 relative to the cylindrical inner portion 13a of the cylindrical wall portion 13 of the insertion opening attachment 11. As a result, it is possible to detect an insertion state of the endoscope insertion unit 2 that the endoscope insertion unit 2 is inserted in the cylindrical inner portion 13a of the cylindrical wall portion 13 of the insertion opening attachment 11.

FIG. 1 shows a state that the endoscope insertion unit 2 is inserted in the cylindrical inner portion 13a of the cylindrical wall portion 13 of the insertion opening attachment 11. At this time, since part of light emitted from the light emitting element 17a is blocked by the endoscope insertion unit 2, a light volume that enters the light receiving element 17 is reduced. As a result, it is possible to confirm that the endoscope insertion unit 2 is inserted in the cylindrical inner portion 13a of the insertion opening attachment 11.

It is to be noted the insertion state detection unit 15 is not restricted to the pair of light receiving/emitting elements, and generally known various techniques can be used as long as they can be disposed to the insertion opening attachment 11.

The attachment state detection unit 16 detects whether the insertion opening attachment 11 is attached to the human mouth H1. The attachment state detection unit 16 is formed of a second light receiving element 18 arranged on an outer peripheral wall surface of the cylindrical wall portion 13 of the insertion opening attachment 11. The second light receiving element 18 is provided on the outer wall surface of the cylindrical wall portion 13 at a position where it is in contact with an inner surface of the human mouth H1 or surrounded by the living body H when the insertion opening attachment 11 is disposed to the human mouth H1.

FIG. 2 is a view showing a state that the insertion opening attachment 11 is not attached to the human mouth H1. That is, the second light receiving element 18 is exposed to the outside and irradiated with outside light.

On the other hand, as shown in FIG. 1 and FIG. 3, in a state that the insertion opening attachment 11 is attached to the human mouth H1, a majority of the outside light is blocked by the living body H. Therefore, the second light receiving element 18 is not irradiated with the majority of outside light or, even if it is irradiated, an amount of outside light is very small, and a light volume of the light detected by the second light receiving element 18 is smaller than that detected in a state that the insertion opening attachment 11 is not attached to the human mouth H1. Therefore, whether the insertion opening attachment 11 is attached to the human mouth H1 can be detected based on the light volume of outside light applied to the second light receiving element 18.

It is to be noted that, when the insertion opening attachment 11 is made of a material that blocks light in Δλ that is a wavelength region of part of the outside light, arranging a filter that allows only light in the same wavelength region Δλ to pass therethrough on a light receiving surface of the second light receiving element 18 is desirable.

As shown in FIG. 5, the light receiving element 17b of the insertion state detection unit 15 and the second light receiving element 18 of the attachment state detection unit 16 are connected to, e.g., a control circuit 19 arranged in the insertion opening attachment 11. A battery 20 and a transmitter 21 are further connected to the control circuit 19. The transmitter 21 is connected to an endoscopic system 22 through a cable or in a wireless manner. Further, a detection status of the insertion state detection unit 15 and a detection status of the attachment state detection unit 16 are transmitted from the transmitter 21 to the endoscopic system 22 through a wire or in a wireless manner.

The endoscopic system 22 has an insertion state determination unit 23. The insertion state determination unit 23 in the endoscopic system 22 determines a positional relationship between the insertion unit 2 of the endoscope 1 and a living body opening portion (the human mouth H1) from a detection result of the insertion state detection unit 15 and a detection result of the attachment state detection unit 16. Here, the insertion state determination unit 23 determines that the insertion unit 2 is inserted in the human mouth H1 when the insertion state detection unit 15 detects that the insertion unit 2 is inserted in the insertion opening attachment 11 and the attachment state detection unit 16 detects that the insertion opening attachment 11 is attached to the insertion opening.

It is to be noted that the control circuit 19, the battery 20, and the transmitter 21 connected to the insertion state detection unit 15 may be different from the control circuit 19, the battery 20, and the transmitter 21 connected to the detection unit of the attachment state detection unit 16.

Moreover, the control circuit 19, the battery 20, and the transmitter 21 may be arranged outside the insertion opening attachment 11, and these components may be connected to the insertion opening attachment 11 through a cable or in a wireless manner. Additionally, the insertion state determination unit 23 may be arranged in the insertion opening attachment 11.

A function of the insertion opening attachment according to this embodiment will now be described.

When the endoscope 1 is used, the insertion opening attachment 11 according to this embodiment is used in combination with the endoscope 1. The insertion opening attachment 11 according to this embodiment is attached in a state that the cylindrical wall portion 13 is inserted in the human mouth H1 as shown in FIG. 3. At this time, the cylindrical wall portion 13 is set to be inserted in the human mouth H1 until the stopper portion 14 abuts on the peripheral edge region of the human mouth H1.

When the insertion opening attachment 11 is correctly disposed in a state that it is inserted in the human mouth H1, the second light receiving element 18 is placed in the human mouth H1 which is a living body opening. Therefore, a light volume of outside light, e.g., illumination light in an examination room that is received by the second light receiving element 18 is smaller than that when the insertion opening attachment 11 is not attached to the human mouth H1.

That is, as shown in FIG. 2, in a state that the insertion opening attachment 11 is disposed to the human mouth H1, the second light receiving element 18 is exposed to the outside and irradiated with the outside light. Therefore, a light volume of the outside light applied to the second light receiving element 18 increases.

On the other hand, when the insertion opening attachment 11 is attached to the human mouth H1, a majority of the outside light is blocked by the living body H. Therefore, the second light receiving element 18 is not irradiated with a majority of the outside light or, even if it is irradiated, an amount of the outside light is very small, and a light volume of the outside light received by the second light receiving element 18 decreases. Therefore, detecting that the light volume of the outside light applied to the second light receiving element 18 has decreased enables detecting that the insertion opening attachment 11 is attached to the human mouth H1.

Further, in this embodiment, the insertion state detection unit 15 can detect an insertion state indicative of whether the endoscope insertion unit 2 is inserted in the cylindrical inner portion 13a of the cylindrical wall portion 13 of the insertion opening attachment. That is, as shown in FIG. 3, in a state that the endoscope insertion unit 2 is not inserted in the cylindrical inner portion 13a of the cylindrical wall portion 13 of the insertion opening attachment 11, the light emitted from the light emitting element 17a of the pair of light receiving/emitting elements is directly detected by the light receiving element 17b.

Therefore, a high light volume that enters the light receiving element 17b is held. Contrary, as shown in FIG. 1, when the endoscope insertion unit 2 is inserted in the cylindrical inner portion 13a of the cylindrical wall portion 13 of the insertion opening attachment 11, part of the light emitted from the light emitting element 17a is blocked by the endoscope insertion unit 2, and hence a light volume that enters the light receiving element 17b decreases.

As a result, it can be confirmed that the endoscope insertion unit 2 is inserted in the cylindrical inner portion 13a of the cylindrical wall portion 13 of the insertion opening attachment 11.

Moreover, a detection status of the insertion state detection unit 15 and a detection status of the attachment state detection unit 16 are input to the control circuit 19, and then they are transmitted from the transmitter 21 to the endoscopic system 22 through a wire or in a wireless manner. Additionally, the insertion state determination unit 23 of the endoscopic system 22 determines that the insertion unit 2 is inserted in the human mouth H1 when the insertion state detection unit 15 detects that the insertion unit 2 is inserted in the insertion opening attachment 11 and the attachment state detection unit 16 detects that the insertion opening attachment 11 is attached to the insertion opening.

Thus, the insertion opening attachment according to the embodiment exercises the following effect. That is, the insertion opening attachment 11 has two detection units, i.e., the insertion state detection unit 15 and the attachment state detection unit 16. Therefore, the attachment state detection unit 16 can detect an attachment state indicative of whether the insertion opening attachment 11 is attached to the human mouth H1, and the insertion state detection unit 15 can detect an insertion state indicative of whether the endoscope insertion unit 2 is inserted in the cylindrical inner portion 13a of the cylindrical wall portion 13 of the insertion opening attachment 11. The insertion state of the endoscope insertion unit 2 and the attachment state of the insertion opening attachment 11 can be independently detected by the insertion state detection unit 15 and the attachment state detection state 16, respectively.

Further, when the insertion state detection unit 15 detects that the insertion unit 2 is inserted in the insertion opening attachment 11 and the attachment state detection unit 16 detects that the insertion opening attachment 11 is attached to the insertion opening, the insertion state determination unit 23 can correctly determine that the insertion unit 2 is inserted in the human mouth H1.

As a result, a state that the endoscope insertion unit 2 alone is inserted in the insertion opening attachment 11 but the insertion opening attachment 11 is not attached to the living body, i.e., a state that the endoscope insertion unit 2 is not actually inserted in the living body opening portion is not erroneously detected as a state that the endoscope insertion unit 2 is inserted in the living body opening portion.

Therefore, for example, like a case where illumination light is emitted from the light source apparatus 7 of the endoscope 1 when the endoscope insertion unit 2 is not actually inserted in the living body opening portion, it is possible to prevent unnecessary light from being applied to the outside of the living body opening, reduce energy loss, or avoid an unpleasant situation, e.g., application of intensive illumination light to eyes. As a result, it is possible to provide the insertion opening attachment 11 that can assuredly detect the inside or the outside of a body without erroneous detection.

Further, in this embodiment, the second light receiving element 18 of the attachment state detection unit 16 is assuredly provided on the outer wall surface of the cylindrical wall portion 13 placed in the human mouth H1 in the attached state that the insertion opening attachment 11 is attached to the human mouth H1. Therefore, the second light receiving element 18 can assuredly detect the attachment state that the insertion opening attachment 11 is attached to the human mouth H1.

Furthermore, the light emitting element 17a of the insertion state detection unit 15 is arranged on the inner peripheral surface of the cylindrical inner portion 13a of the cylindrical wall portion 13, and the second light receiving element 18 is provided on the outer wall surface of the cylindrical wall portion 13. Moreover, since the cylindrical wall portion 13 of the insertion state detection unit 15 has properties that block at least part of outside light or the like, an amount of light from the light emitting element 17a that passes through the cylindrical wall portion 13 and leaks to the outer wall surface side of the cylindrical inner portion 13a of the cylindrical wall portion 13 is small, and hence the second light receiving element 18 is hardly affected by the light emitted from the light emitting element 17a.

It is to be noted that the example where the insertion opening attachment 11 has a shape that is a combination of the cylindrical wall portion 13 formed into a cylindrical shape and the stopper portion 14 formed into a discoid shape has been described for simplicity in this embodiment, but the insertion opening attachment 11 is not restricted thereto. Actually, in case of disposing the insertion opening attachment 11 to, e.g., the human mouth H1, it can be designed into a shape that can fit to an oral cavity while considering positions and sizes of teeth, lips, and others.

### [Modification of First Embodiment]

In the insertion opening attachment 11 according to the first embodiment, the configuration where the second light receiving element 18 as the attachment state detection unit 16 is provided on the outer wall surface of the cylindrical shape wall portion 13 formed into a cylindrical shape in the insertion opening attachment 11 has been described, but a second light receiving element 18 is provided on a surface of a discoid stopper portion 14 where a cylindrical wall portion 13 is arranged in an insertion opening attachment 31 according to this modification as shown in FIG. 6.

With this configuration, since the second light receiving element 18 is not inserted into an oral cavity of a human mouth H1, a risk that the second light receiving element 18 is damaged when teeth come into contact with the second light receiving element 18 is diminished. Further, since the second light receiving element 18 is hard to be exposed to a liquid such as saliva, a waterproof configuration of the second light receiving element 18 for avoiding penetration of the liquid can be simplified.

### [Second Embodiment]

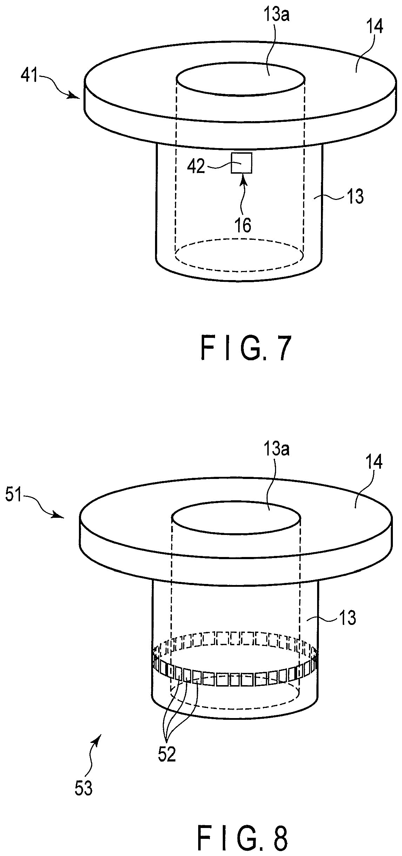

FIG. 7 shows a second embodiment according to the present invention. This embodiment is obtained by changing the configuration of the insertion opening attachment 11 in the first embodiment (see FIG. 1 to FIG. 5) as follows. It is to be noted that like reference numerals denote parts equal to those in FIG. 1 to FIG. 5 and a description thereof will be omitted.

A difference lies in that a temperature sensor 42 is provided in an insertion opening attachment 41 according to this embodiment in place of the second light receiving element 18 which is the attachment state detection unit 16 in the first embodiment. The temperature sensor 42 is arranged at a position where it comes into contact with a living body when the insertion opening attachment 41 is attached to a living body opening. For example, as shown in FIG. 7, the temperature sensor 42 is arranged on an outer wall surface of a cylindrical wall portion formed into a cylindrical shape in the insertion opening attachment 41.

Further, in case of a mouthpiece that the insertion opening attachment 41 is disposed to a mouth, it is preferable to arrange the temperature sensor 42 at a position where it comes into contact with lips.

As the temperature sensor 42 according to this embodiment, a temperature sensor having any configuration can be used irrespective of a contact type or a contactless type. It is to be noted that, in case of using a contact type temperature sensor, whether the insertion opening attachment 41 is attached to the human mouth H1 can be more assuredly detected. Furthermore, in case of using a contactless type temperature sensor, even before the insertion opening attachment 41 is inserted into an oral cavity of the human mouth H1, a temperature of a living body is detected, an attached state may be possibly erroneously detected, and hence using the contact type temperature sensor is desirable.

It is to be noted that, as an insertion state detection unit 15, a type which is the same as the first embodiment or generally known various types can be used, and hence a description thereof will be omitted.

A function of the configuration will now be described. A temperature of a general living body is in the range of approximately 35°C to 40°C. When the insertion opening attachment 41 according to this embodiment is attached to an opening of the living body, arranging the temperature sensor 42 at a position where it can assuredly come into contact with the living body enables securely detecting a temperature of the living body.

Therefore, when the insertion opening attachment 41 according to this embodiment is normally attached to the living body, providing the temperature sensor 42 at a position where it can assuredly come into contact with the living body enables securely detecting that the insertion opening attachment 41 is normally arranged to the living body with use of the temperature sensor 42. In order words, it is possible to reduce a risk of erroneous detection caused due to determining attachment to the living body by the attachment state detection unit 16 when the insertion opening attachment 41 is not normally attached to the living body.

Thus, in addition to the effect of the first embodiment, this embodiment can exercise an effect that erroneous detection can be avoided in the insertion opening attachment 41 according to this embodiment even in an environment where the insertion opening attachment 41 is not irradiated with sufficient outside light due to brightness or a position of indoor lighting, a positional relationship relative to an operator, and others.

### [Modification of Second Embodiment]

The description has been given as to the configuration where, in the insertion opening attachment 41 according to the second embodiment (see FIG. 7), one temperature sensor 42 which is the attachment state detection unit 16 is provided on the outer wall surface of the cylindrical wall portion 13 formed into the cylindrical shape in the insertion opening attachment 41. On the other hand, in an insertion opening attachment 51 according to this modification, as shown in FIG. 8, temperature sensors 52 (which will be referred to as a temperature sensor group 53 hereinafter) are arranged to be aligned on an outer wall surface of a cylindrical wall portion 13 having a cylindrical shape along a circumferential direction.

According to this modification, when an operator attaches the insertion opening attachment 51 according to this modification to an opening portion of a living body, there is almost no possibility that the operator accidentally touches all the temperature sensors 52 on the outer wall surface of the cylindrical wall portion 13 at the same time. Therefore, it is possible to avoid erroneous detection that occurs when hands or fingers of the operator touch the temperature sensors 52.

On the other hand, when the insertion opening attachment 51 is attached to the living body, all the temperature sensors 52 indicate substantially the same temperatures, and hence the attachment to the living body can be more assuredly detected. In this modification, the temperature sensors 52 can be used irrespective of a contact type or a contactless type.

In case of the contactless type, when the insertion opening attachment 51 is attached to the opening of the living body, since all the temperature sensors 52 detect a temperature of the living body, an attachment state of the insertion opening attachment 51 can be more assuredly detected. Therefore, using the contactless type temperature sensor is desirable. It is to be noted that, in the contact type temperature sensors, the contacting temperature sensors and the non-contacting temperature sensors may possibly detect different temperatures, and hence correction may be required in some situations.

### [Third Embodiment]

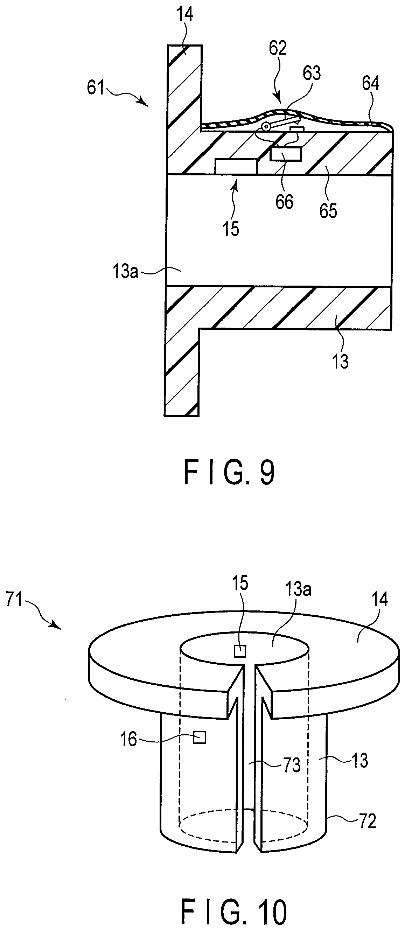

FIG. 9 shows a third embodiment according to the present invention. This embodiment is obtained by changing the configuration of the insertion opening attachment 11 according to the first embodiment (see FIG. 1 to FIG. 5) as follows. It is to be noted that, in FIG. 9, like reference numerals denote parts equal to those in FIG. 1 to FIG. 5, thereby omitting a description thereof.

An insertion opening attachment 61 according to this embodiment is different from the first embodiment in that an electrical function element 62 that allows electrical resistance to change in response to a pressure from a living body is provided in place of the second light receiving element 18 which is the attachment state detection unit 16 in the first embodiment. The electrical function element 62 in this embodiment is a switch that changes over an insulating state and a conducting state in response to stress from the living body when the insertion opening attachment 61 is attached to the living body.

FIG. 9 is a longitudinal cross-sectional view of the insertion opening attachment 61 according to this embodiment. The switch 63 is disposed to an outer wall surface of a cylindrical wall portion having a cylindrical shape of the insertion opening attachment 61. The switch 63 is disposed at a position where a pressure from the living body is applied in a state that the insertion opening attachment 61 is attached to the living body. For example, the switch 63 is arranged at a position where it comes into contact with lips, teeth, or gums.

Here, when the switch 63 is arranged at a position where a pressure is applied by lips, there is less chance that an excessive pressure is applied to the switch 63 or a pressure is locally concentrated, which is preferable.

A waterproof cover 64 is provided on an outer side of the switch 63. As a result, it prevents a body fluid such as saliva from entering the switch 63. It is to be noted that the waterproof cover 64 is configured to prevent saliva or the like from entering and desirably made of a material that is not easily damaged by teeth and others.

Further, the switch 63 is adjusted so that a state can be changed over in response to a pressure that is close to a pressure applied by a subject without being conscious of this application in particular when the insertion opening attachment 61 is attached to a mouth. The switch 63 is connected to a detection circuit 66 arranged in an attachment main body 65 of the endoscope insertion opening attachment 61. When the switch 63 is turned on, the detection circuit 66 determines that the insertion opening attachment 61 is attached to a living body opening portion and transmits a detection state from a transmitter 21 (see FIG. 5) to an endoscopic system 22 (see FIG. 5).

A function of the configuration will now be described. In this embodiment, the switch 63 is turned on and an attachment state of the insertion opening attachment 61 can be detected from a pressure naturally produced when the insertion opening attachment 61 is attached to the opening portion of the living body.

Thus, according to this embodiment, in addition to the effect of the first embodiment, the switch 63 is turned on and an attachment state of the insertion opening attachment 61 is detected in response to only a pressure that acts on the attachment main body 65 of the insertion opening attachment 61 when the insertion opening attachment 61 is attached to the opening portion of the living body, and hence erroneous detection does not occur due to a change in outside light or in temperature.

### [Other Embodiments]

It is to be noted that, as an observation target in each of the foregoing embodiments, the example of the insertion opening attachment 11, 31, 41, 51, or 61 attached to the mouth has been described, but the present invention is not restricted thereto. For example, it is possible to use the present invention as an insertion opening attachment for an endoscope or the like configured to observe a respiratory organ such as a lower digestive endoscope, a laparoscope, or a bronchoscope typified by a large intestine endoscope. Moreover, the present invention can be used as an insertion opening attachment for an endoscope for animals that is used for farm animals or pets or an industrial endoscope configured to observe the inside of an engine or the like. In such a case, the insertion opening attachment is appropriately selected in accordance with an observation target.

For example, like an insertion opening attachment 71 according to a fourth embodiment shown in FIG. 10, it is possible to provide a notch portion 73 extended in a center line direction of a cylindrical wall portion 13 having a cylindrical shape in an attachment main body 72. In this case, when an opening width of the notch portion 73 is adjusted in accordance with a thickness of an endoscope insertion unit 2 to be used or a size of the insertion opening, a diameter of the cylindrical wall portion 13 having the cylindrical shape 13 in the attachment main body 72 of the insertion opening attachment 71 can be adjusted.

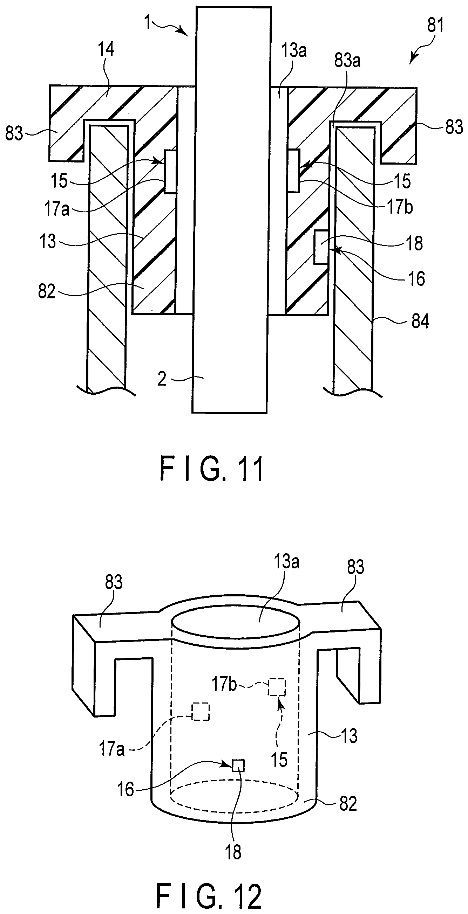

Additionally, it is possible to configure the insertion opening attachment like an insertion opening attachment 81 according to a fifth embodiment shown in FIG. 11 and FIG. 12. Here, one or more hook portions 83 are provided at one end of a cylindrical wall portion 13 having a cylindrical shape in an attachment main body 82 of the insertion opening attachment 81, and the hook portions 83 are disposed to an end edge region of an opening portion 84a at one end of a tubular body 84 of an observation target.

The insertion opening attachment according to each of the foregoing embodiments is an insertion opening attachment that is used in combination with the endoscope apparatus configured to observe an inner surface of an object of a subject and attached to an insertion opening which is an opening portion of the object, the insertion opening attachment has the attachment main body having the cylindrical shape fixed to the peripheral edge region of the opening portion of the insertion opening of the object while being inserted in the insertion opening, and the attachment main body has the attachment state detection unit that detects whether the attachment main body is attached to the insertion opening and the insertion state detection unit which detects whether the insertion opening is inserted in the insertion opening.

The insertion opening attachment according to each of the foregoing embodiments has the following characteristics.
(1) In the state that the attachment main body is attached to the insertion opening, the attachment state detection unit is formed on the surface of the attachment main body facing the object, and the insertion state detection unit is formed on the surface facing the insertion unit when the insertion unit is inserted into the attachment main body.
(2) The attachment main body has the cylindrical wall portion inserted into the insertion opening and the stopper portion that regulates falling of the cylindrical wall portion to the inside of the object.
(3) The attachment state detection unit has a photodetector, and it detects an attachment state of the attachment main body by utilizing a change in light volume entering the photodetector caused in response to the attachment state of the attachment main body.
(4) The attachment main body has the cylindrical wall portion including the opening into which the insertion unit can be inserted, and the photodetector is arranged on the outer surface of the cylindrical wall portion and detects an attachment state of the attachment main body by utilizing a reduction in light volume entering the photodetector when outside light is blocked by the object in a state that the attachment main body is attached to the insertion opening portion.
(5) The stopper portion is provided at one end of the cylindrical wall portion and made of the plate-like extended member that is extended toward the outer side in the direction crossing the inserting direction of the insertion unit.
(6) The attachment state detection unit has the photodetector, and the photodetector is provided on the surface of the extended member facing the object in a state that the attachment main body is attached to the insertion opening.
(7) The attachment state detection unit has the temperature sensor.
(8) The temperature sensor is a contact type temperature sensor, and the detection unit of the temperature sensor is arranged at a position where it comes into contact with the object in a state that the attachment main body is attached to the insertion opening portion.
(9) The attachment state detection unit has the temperature sensor group including the temperature sensors, the respective temperature sensors constituting the temperature sensor group are the contactless type sensors, and all the temperature sensors are arranged at positions where they face the object in a state that the attachment main body is attached to the insertion opening portion.
(10) The attachment state detection unit has the electrical function element that allows electrical resistance to change in accordance with a pressure, a portion of the electrical function element that detects a pressure is arranged at a position that is in contact with the object in a state that the attachment main body is attached to the insertion opening portion.
(11) The electrical function element is a switch element that changes over a conducting state and an insulating state in accordance with a pressure.
(12) The switch element is covered with the waterproof coating.
(13) The attachment main body has the insertion state determination unit that determines a positional relationship between the insertion unit and the insertion opening of the object based on an output from the insertion state detection unit and an output from the attachment state detection unit.
(14) The insertion state determination unit determines that the insertion unit is inserted in the insertion opening when the insertion state detection unit detects that the insertion unit is inserted and the attachment state detection unit detects the attachment to the insertion opening.

Additionally, the present invention is not restricted to the foregoing embodiments, and it can be modified in many ways. Accordingly, various modifications may be made without departing from the scope of the general inventive concept as defined by the appended claims and their equivalents.

## Claims

1. An insertion opening attachment (11; 41; 51; 61; 71; 81) that is used in combination with an endoscope apparatus (1) configured to observe the inside of a subject and that is attached to an insertion opening (H1) opened in the subject into which an insertion unit (2) of the endoscope apparatus (1) is inserted,
wherein the insertion opening attachment (11; 41; 51; 61; 71; 81) comprises:
an attachment main body (12; 72) having a flanged cylindrical shape which is fixed to a peripheral edge region of the insertion opening (H1) in a state the insertion opening attachment (11; 41; 51; 61; 71; 81) is inserted in the insertion opening (H1), and
the attachment main body (12; 72) comprises:
an attachment state detection unit (16; 42; 62) which detects whether the attachment main body (12; 72) is attached to the insertion opening (H1);
wherein the attachment main body (12; 72) furthermore comprises an insertion state detection unit (15) which detects whether the insertion unit (2) of the endoscope apparatus (1) is inserted in the insertion opening (H1), and
wherein the attachment main body (12; 72) comprises an insertion state determination unit (23) which determines a positional relationship between the insertion unit (2) and the insertion opening (H1) of the object based on an output from the insertion state detection unit (15) and an output from the attachment state detection unit (16; 42; 62), **characterised in that** said insertion state determination unit (23) determines that the insertion unit (2) is inserted in the insertion opening (H1) when the insertion state detection unit (15) detects that the insertion unit (2) is inserted and the attachment state detection unit (16; 42; 62) detects the attachment to the insertion opening (H1).

2. The insertion opening attachment (11; 41; 51; 61; 71; 81) according to claim 1,
**characterized in that** a state that the attachment main body (12; 72) is attached to the insertion opening (H1),
the attachment state detection unit (16; 42; 62) is arranged on a surface of the attachment main body (12; 72) facing the subject, and
the insertion state detection unit (15) is arranged on a surface facing the insertion unit (2) when the insertion unit (2) is inserted in the attachment main body (12; 72).

3. The insertion opening attachment (11; 41; 51; 61; 71; 81) according to claim 2,
**characterized in that** the attachment main body (12; 72) comprises:
a cylindrical wall portion (13) which is inserted into the insertion opening (H1); and
a stopper portion (14) which locks at an opening periphery of the insertion opening (H1) and prevents the cylindrical wall portion (13) from falling into the insertion opening (H1).

4. The insertion opening attachment (11; 41; 51; 61; 71; 81) according to claim 1,
**characterized in that** the attachment state detection unit (16; 42; 62) comprises a photodetector (18), and
the attachment state detection unit (16; 42; 62) detects an attachment state of the attachment main body (12; 72) by utilizing a change in light volume entering the photodetector (18) caused in accordance with the attachment state of the attachment main body (12; 72).

5. The insertion opening attachment (11; 41; 51; 61; 71; 81) according to claim 4,
**characterized in that** the attachment main body (12; 72) comprises a cylindrical wall portion (13) comprising an opening which allows the insertion unit (2) to be inserted therethrough,
the photodetector (18) is arranged on an outer surface of the cylindrical wall portion (13), and
the photodetector (18) detects an attachment state of the attachment main body (12; 72) by utilizing a reduction in light volume entering the photodetector caused when outside light is blocked by the subject in a state that the attachment main body (12; 72) is attached to the insertion opening (H1).

6. The insertion opening attachment (11; 41; 51; 61; 71; 81) according to claim 3,
**characterized in that** the stopper portion (14) is formed of a plate-like extended member which is provided at one end of the cylindrical wall portion (13) and extended toward the outer side in a direction crossing an inserting direction of the insertion unit (2).

7. The insertion opening attachment (11; 41; 51; 61; 71; 81) according to claim 6,
**characterized in that** the attachment state detection unit (16; 42; 62) comprises a photodetector, and
the photodetector is provided on a surface of the extended member facing the object in a state that the attachment main body (12; 72) is attached to the insertion opening (H1).

8. The insertion opening attachment (11; 41; 51; 61; 71; 81) according to claim 1,
**characterized in that** the attachment state detection unit (16; 42; 62) comprises a temperature sensor.

9. The insertion opening attachment (11; 41; 51; 61; 71; 81) according to claim 8,
**characterized in that** the temperature sensor is a contact type temperature sensor, and
a detection unit of the temperature sensor is arranged at a position where it comes into contact with the subject in a state that the attachment main body (12; 72) is attached to the insertion opening (H1).

10. The insertion opening attachment (11; 41; 51; 61; 71; 81) according to claim 1,
**characterized in that** the attachment state detection unit (16; 42; 62) comprises a temperature sensor group comprising temperature sensors,
each of the temperature sensors constituting the temperature sensor group is a contactless type sensor, and
all the temperature sensors are arranged at positions where they face the subject in a state that the attachment main body (12; 72) is attached to the insertion opening (H1).

11. The insertion opening attachment (11; 41; 51; 61; 71; 81) according to claim 1,
**characterized in that** the attachment state detection unit (16; 42; 62) comprises a electrical function element (62) which allows electrical resistance to change in response to a pressure, and
a portion of the electrical function element (62) which detects a pressure is arranged at a portion where it comes into contact with the subject in a state that the attachment main body (12; 72) is attached to the insertion opening (H1).

12. The insertion opening attachment (11; 41; 51; 61; 71; 81) according to claim 11,
**characterized in that** the electrical function element (62) is a switch element (63) which changes over a conducting state and an insulating state in response to a pressure.

13. The insertion opening attachment (11; 41; 51; 61; 71; 81) according to claim 12,
**characterized in that** the switch element (63) is covered with a waterproof coating (64).

## Patentansprüche

1. Einführöffnung-Ansatz (11; 41; 51; 61; 71; 81), welcher in Kombination mit einer Endoskopvorrichtung (1) verwendet wird, mit welchem das Innere einer Person beobachtbar ist und welcher an einer Einführöffnung (H1) angesetzt wird, die sich in die Person öffnet und in welche eine Einführeinheit (2) der Endoskopvorrichtung (1) eingeführt wird,
wobei der Einführöffnung-Ansatz (11; 41; 51; 61; 71; 81) aufweist:
einen Ansatzhauptkörper (12; 72) der eine mit einem Flansch versehene zylindrische Form hat, und der an einem Umfangsrandbereich der Einführöffnung (H1) in einem Zustand befestigt ist, in welchem der Einführöffnung-Ansatz (11; 41; 51; 61; 71; 81) in die Einführöffnung (H1) eingeführt ist, und
wobei der Ansatzhauptkörper (12; 72) aufweist:
eine Ansatzzustanderkennungseinheit (16; 42; 62), die erkennt, ob der Ansatzhauptkörper (12; 72) an die Einführöffnung (H1) angesetzt ist;
wobei der Ansatzhauptkörper (12; 72) weiterhin eine Einführzustanderkennungseinheit (15) aufweist, die erkennt, ob die Einführeinheit (2) der Endoskopvorrichtung (1) in die Einführöffnung (H1) eingeführt ist, und
wobei der Ansatzhauptkörper (12; 72) eine Einführzustandbestimmungseinheit (23) aufweist, welche eine Lagebeziehung zwischen der Einführeinheit (2) und der Einführöffnung (H1) des Objekts auf der Grundlage eines Ausgangs von der Einführzustanderkennungseinheit (15) und eines Ausgangs von der Ansatzzustanderkennungseinheit (16; 42; 62) bestimmt,
**dadurch gekennzeichnet, dass**
die Einführzustandbestimmungseinheit (23) bestimmt, dass die Einführeinheit (2) in die Einführöffnung (H1) eingeführt ist, wenn die Einführzustanderkennungseinheit (15) erkennt, dass die Einführeinheit (2) eingeführt ist, und die Ansatzzustanderkennungseinheit (16; 42; 62) das Ansetzen an der Einführöffnung (H1) erkennt.

2. Einführöffnung-Ansatz (11; 41; 51; 61; 71; 81) nach Anspruch 1, **dadurch gekennzeichnet, dass** ein Zustand, dass der Ansatzhauptkörper (12; 72) an der Einführöffnung (H1) angesetzt ist,
die Ansatzzustanderkennungseinheit (16; 42; 62) an einer Oberfläche des Ansatzhauptkörpers (12; 72) angeordnet ist, die zu der Person zeigt, und
die Einführzustanderkennungseinheit (15) an einer Oberfläche angeordnet ist, welche zu der Einführeinheit (2) zeigt, wenn die Einführeinheit (2) in den Ansatzhauptkörper (12; 72) eingeführt ist.

3. Einführöffnung-Ansatz (11; 41; 51; 61; 71; 81) nach Anspruch 2, **dadurch gekennzeichnet, dass** der Ansatzhauptkörper (12; 72) aufweist:
einen zylindrischen Wandabschnitt (13), der in die Einführöffnung (H1) eingeführt ist; und
einen Stopperabschnitt (14), der an einem Öffnungsumfang der Einführöffnung (H1) verriegelt ist und verhindert, dass der zylindrische Wandabschnitt (13) in die Einführöffnung (H1) fällt.

4. Einführöffnung-Ansatz (11; 41; 51; 61; 71; 81) nach Anspruch 1, **dadurch gekennzeichnet, dass** die Ansatzzustanderkennungseinheit (16; 42; 62) einen Fotodetektor (18) aufweist, und
die Ansatzzustanderkennungseinheit (16; 42; 62) einen angesetzten Zustand des Ansatzhauptkörpers (12; 72) erkennt, indem eine Änderung des Lichtvolumens verwendet wird, welches in den Fotodetektor (18) eintritt und entsprechend des angesetzten Zustands des Ansatzhauptkörper (12; 72) verursacht wird.

5. Einführöffnung-Ansatz (11; 41; 51; 61; 71; 81) nach Anspruch 4, **dadurch gekennzeichnet, dass** der Ansatzhauptkörper (12; 72) einen zylindrischen Wandabschnitt (13) aufweist, der eine Öffnung hat, welche das Einführen der Einführeinheit (2) hierdurch erlaubt,
der Fotodetektor (18) an einer äußeren Oberfläche des zylindrischen Wandabschnitts (13) angeordnet ist, und
der Fotodetektor (18) einen angesetzen Zustand des Ansatzhauptkörpers (12; 72) erkennt, indem eine Verringerung des Lichtvolumens verwendet wird, welches in den Fotodetektor eintritt und verursacht wird, wenn Licht von außen von der Person in einem Zustand blockiert wird, in welchem der Ansatzhauptkörper (12; 72) an die Einführöffnung (H1) angesetzt ist.

6. Einführöffnung-Ansatz (11; 41; 51; 61; 71; 81) nach Anspruch 3, **dadurch gekennzeichnet, dass** der Stopperabschnitt (14) aus einem plattenförmigen Verlängerungsteil gebildet ist, das an einem Ende des zylindrischen Wandabschnitts (13) angeordnet ist, und sich in Richtung der Außenseite in einer Richtung erstreckt, welche die Einführrichtung der Einführeinheit (2) kreuzt.

7. Einführöffnung-Ansatz (11; 41; 51; 61; 71; 81) nach Anspruch 6, **dadurch gekennzeichnet, dass** die Ansatzzustanderkennungseinheit (16; 42; 62) einen Fotodetektor aufweist, und
der Fotodetektor an einer Oberfläche des Verlängerungsbauteils und zu dem Objekt in einem Zustand zeigend angeordnet ist, in welchem der Ansatzhauptkörper (12; 72) an der Einführöffnung (H1) angesetzt ist.

8. Einführöffnung-Ansatz (11; 41; 51; 61; 71; 81) nach Anspruch 1, **dadurch gekennzeichnet, dass** die Ansatzzustanderkennungseinheit (16; 42; 62) einen Temperatursensor aufweist.

9. Einführöffnung-Ansatz (11; 41; 51; 61; 71; 81) nach Anspruch 8, **dadurch gekennzeichnet, dass** der Temperatursensor ein Temperatursensor vom Kontakttyp ist, und
eine Erkennungseinheit des Temperatursensors an einer Position angeordnet ist, wo sie in Kontakt mit der Person in einem Zustand gelangt, in welchem der Ansatzhauptkörper (12; 72) an die Einführöffnung (H1) angesetzt ist.

10. Einführöffnung-Ansatz (11; 41; 51; 61; 71; 81) nach Anspruch 1, **dadurch gekennzeichnet, dass** die Ansatzzustanderkennungseinheit (16; 42; 62) eine Temperatursensorgruppe bestehend aus Temperatursensoren aufweist,
jeder der Temperatursensoren, welche die Temperatursensorgruppe bilden, ein Sensor vom kontaktlosen Typ ist, und
alle Temperatursensoren an Positionen angeordnet sind, wo sie zu der Person in einem Zustand zeigen, in welchem der Ansatzhauptkörper (12; 72) an die Einführöffnung (H1) angesetzt ist.

11. Einführöffnung-Ansatz (11; 41; 51; 61; 71; 81) nach Anspruch 1, **dadurch gekennzeichnet, dass** die Ansatzzustanderkennungseinheit (16; 42; 62) ein elektrisches Funktionselement (62) aufweist, welches eine elektrische Widerstandsänderung in Reaktion auf Druck erlaubt, und
ein Abschnitt des elektrisches Funktionselements (62), der einen Druck erkennt, an einem Abschnitt angeordnet ist, der in Kontakt mit der Person in einen Zustand gelangt, in welchem der Ansatzhauptkörper (12; 72) an die Einführöffnung (H1) angesetzt ist.

12. Einführöffnung-Ansatz (11; 41; 51; 61; 71; 81) nach Anspruch 11, **dadurch gekennzeichnet, dass** das elektrische Funktionselement (62) ein Schalterelement (63) ist, welches in Reaktion auf einen Druck zwischen einem leitfähigen Zustand und einem isolierten Zustand umschaltet.

13. Einführöffnung-Ansatz (11; 41; 51; 61; 71; 81) nach Anspruch 12, **dadurch gekennzeichnet, dass** das Schalterelement (63) mit einem wasserdichten Überzug (64) bedeckt ist.

## Revendications

1. Attache d'ouverture d'insertion (11 ; 41 ; 51 ; 61 ; 71 ; 81) qui est utilisée en combinaison avec un appareil à endoscope (1) configuré pour observer l'intérieur d'un sujet et qui est attachée à une ouverture d'insertion (H1) ouverte dans le sujet dans lequel une unité d'insertion (2) de l'appareil à endoscope (1) est insérée,
dans laquelle l'attache d'ouverture d'insertion (11 ; 41 ; 51 ; 61 ; 71 ; 81) comprend :
un corps principal d'attache (12 ; 72) ayant une forme cylindrique bridée qui est fixée à une région de bord périphérique de l'ouverture d'insertion (H1) dans un état où l'attache d'ouverture d'insertion (11 ; 41 ; 51 ; 61 ; 71 ; 81) est insérée dans l'ouverture d'insertion (H1), et
le corps principal d'attache (12 ; 72) comprend :
une unité de détection d'état d'attache (16 ; 42 ; 62) qui détecte si le corps principal d'attache (12 ; 72) est attaché à l'ouverture d'insertion (H1) ;
dans laquelle le corps principal d'attache (12 ; 72) comprend de surcroît une unité de détection d'état d'insertion (15) qui détecte si l'unité d'insertion (2) de l'appareil à endoscope (1) est insérée dans l'ouverture d'insertion (H1), et
dans laquelle le corps principal d'attache (12 ; 72) comprend une unité de détermination d'état d'insertion (23) qui détermine une relation de position entre l'unité d'insertion (2) et l'ouverture d'insertion (H1) de l'objet sur la base d'une sortie de l'unité de détection d'état d'insertion (15) et d'une sortie de l'unité de détection d'état d'attache (16 ; 42 ; 62),
**caractérisée en ce que** ladite unité de détermination d'état d'insertion (23) détermine que l'unité d'insertion (2) est insérée dans l'ouverture d'insertion (H1) lorsque l'unité de détection d'état d'insertion (15) détecte que l'unité d'insertion (2) est insérée et que l'unité de détection d'état d'attache (16 ; 42 ; 62) détecte l'attache à l'ouverture d'insertion (H1).

2. Attache d'ouverture d'insertion (11 ; 41 ; 51 ; 61 ; 71 ; 81) selon la revendication 1,
**caractérisée en ce qu'**un état où le corps principal d'attache (12 ; 72) est attaché à l'ouverture d'insertion (H1),
l'unité de détection d'état d'attache (16 ; 42 ; 62) est agencée sur une surface du corps principal d'attache (12 ; 72) en regard du sujet, et
l'unité de détection d'état d'insertion (15) est agencée sur une surface en regard de l'unité d'insertion (2) lorsque l'unité d'insertion (2) est insérée dans le corps principal d'attache (12 ; 72).

3. Attache d'ouverture d'insertion (11 ; 41 ; 51 ; 61 ; 71 ; 81) selon la revendication 2,
**caractérisée en ce que** le corps principal d'attache (12 ; 72) comprend :
une portion de paroi cylindrique (13) qui est insérée dans l'ouverture d'insertion (H1) ; et
une portion de bouchon (14) qui se verrouille au niveau d'une périphérie d'ouverture de l'ouverture d'insertion (H1) et empêche la portion de paroi cylindrique (13) de tomber dans l'ouverture d'insertion (H1).

4. Attache d'ouverture d'insertion (11 ; 41 ; 51 ; 61 ; 71 ; 81) selon la revendication 1,
**caractérisée en ce que** l'unité de détection d'état d'attache (16 ; 42 ; 62) comprend un photodétecteur (18), et
l'unité de détection d'état d'attache (16 ; 42 ; 62) détecte un état d'attache du corps principal d'attache (12 ; 72) par l'utilisation d'un changement de volume de lumière entrant dans le photodétecteur (18) provoqué en conformité avec l'état d'attache du corps principal d'attache (12 ; 72).

5. Attache d'ouverture d'insertion (11 ; 41 ; 51 ; 61 ; 71 ; 81) selon la revendication 4,
**caractérisée en ce que** le corps principal d'attache (12 ; 72) comprend une portion de paroi cylindrique (13) comprenant une ouverture qui permet à l'unité d'insertion (2) d'être insérée dans celle-ci,
le photodétecteur (18) est agencé sur une surface externe de la portion de paroi cylindrique (13), et
le photodétecteur (18) détecte un état d'attache du corps principal d'attache (12 ; 72) par l'utilisation d'une réduction de volume de lumière entrant dans le photodétecteur provoquée lorsqu'une lumière extérieure est bloquée par le sujet dans un état où le corps principal d'attache (12 ; 72) est attaché à l'ouverture d'insertion (H1).

6. Attache d'ouverture d'insertion (11 ; 41 ; 51 ; 61 ; 71 ; 81) selon la revendication 3,
**caractérisée en ce que** la portion de bouchon (14) est formée d'un organe étendu semblable à une plaque qui est ménagé à une extrémité de la portion de paroi cylindrique (13) et étendu vers le côté externe dans une direction coupant une direction d'insertion de l'unité d'insertion (2).

7. Attache d'ouverture d'insertion (11 ; 41 ; 51 ; 61 ; 71 ; 81) selon la revendication 6,
**caractérisée en ce que** l'unité de détection d'état d'attache (16 ; 42 ; 62) comprend un photodétecteur, et
le photodétecteur est ménagé sur une surface de l'organe étendu en regard de l'objet dans un état où le corps principal d'attache (12 ; 72) est attaché à l'ouverture d'insertion (H1).

8. Attache d'ouverture d'insertion (11 ; 41 ; 51 ; 61 ; 71 ; 81) selon la revendication 1,
**caractérisée en ce que** l'unité de détection d'état d'attache (16 ; 42 ; 62) comprend un capteur de température.

9. Attache d'ouverture d'insertion (11 ; 41 ; 51 ; 61 ; 71 ; 81) selon la revendication 8,
**caractérisée en ce que** le capteur de température est un capteur de température de type à contact, et
une unité de détection du capteur de température est agencée en une position où elle vient en contact avec le sujet dans un état où le corps principal d'attache (12 ; 72) est attaché à l'ouverture d'insertion (H1).

10. Attache d'ouverture d'insertion (11 ; 41 ; 51 ; 61 ; 71 ; 81) selon la revendication 1,
**caractérisée en ce que** l'unité de détection d'état d'attache (16 ; 42 ; 62) comprend un groupe de capteurs de température comprenant des capteurs de température,
chacun des capteurs de température constituant le groupe de capteurs de température est un capteur de type sans contact, et
tous les capteurs de température sont agencés en des positions où ils sont en regard du sujet dans un état où le corps principal d'attache (12 ; 72) est attaché à l'ouverture d'insertion (H1).

11. Attache d'ouverture d'insertion (11 ; 41 ; 51 ; 61 ; 71 ; 81) selon la revendication 1,
**caractérisée en ce que** l'unité de détection d'état d'attache (16 ; 42 ; 62) comprend un élément de fonction électrique (62) qui permet à une résistance électrique de changer en réponse à une pression, et
une portion de l'élément de fonction électrique (62) qui détecte une pression est agencée en une portion où il vient en contact avec le sujet dans un état où le corps principal d'attache (12 ; 72) est attaché à l'ouverture d'insertion (H1).

12. Attache d'ouverture d'insertion (11 ; 41 ; 51 ; 61 ; 71 ; 81) selon la revendication 11,
**caractérisée en ce que** l'élément de fonction électrique (62) est un élément de commutateur (63) qui permute entre un état conducteur et un état isolant en réponse à une pression.

13. Attache d'ouverture d'insertion (11 ; 41 ; 51 ; 61 ; 71 ; 81) selon la revendication 12,
**caractérisée en ce que** l'élément de commutateur (63) est recouvert d'un revêtement étanche à l'eau (64).
